(19) Europäisches Patentamt
European Patent Office
Office européen des brevets

(11) EP 0 926 125 B1

(12) **FASCICULE DE BREVET EUROPEEN**

(45) Date de publication et mention de la délivrance du brevet:
**14.11.2001 Bulletin 2001/46**

(51) Int Cl.[7]: **C07C 51/60**, C07C 53/42, C07C 63/10

(21) Numéro de dépôt: **98403159.1**

(22) Date de dépôt: **15.12.1998**

(54) **Procédé continu de préparation de chlorure de pyvaloyle et de chlorure d'aroyle**

Kontinuierliches Verfahren zur Herstellung von Pivaloylchlorid und Aroylchlorid

Continuous process for the preparation of pyvaloyl chloride and aroyl chloride

(84) Etats contractants désignés:
**BE DE ES FR GB IT**

(30) Priorité: **23.12.1997 FR 9716326**

(43) Date de publication de la demande:
**30.06.1999 Bulletin 1999/26**

(73) Titulaire: **Atofina**
**92800 Puteaux (FR)**

(72) Inventeurs:
- **Ruppin, Christophe**
**69310 Pierre-Benite (FR)**
- **Corbiere, Philippe**
**95470 Survilliers (FR)**

(56) Documents cités:
**US-A- 1 921 767**
**US-A- 1 965 556**
**US-A- 4 163 753**

- **DATABASE WPI Section Ch, Week 8226 Derwent Publications Ltd., London, GB; Class B05, AN 82-53660E XP002077015 & JP 57 082336 A (MITSUBISHI GAS CHEM IND CO LTD), 22 mai 1982**
- **DATABASE WPI Section Ch, Week 8246 Derwent Publications Ltd., London, GB; Class B05, AN 82-98857E XP002077016 & JP 57 165341 A (MITSUBISHI GAS CHEM IND CO LTD) 12 octobre 1982**
- **PATENT ABSTRACTS OF JAPAN vol. 006, no. 165 (C-121), 28 août 1982 & JP 57 082337 A (MITSUBISHI GAS CHEM CO INC), 22 mai 1982**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 926 125 B1

## Description

L'invention concerne un procédé continu de préparation de chlorure de pivaloyle et de chlorure d'aroyle, notamment, de chlorure de benzoyle.

Le chlorure de pivaloyle constitue un intermédiaire de synthèse important de l'industrie chimique. Il est très largement utilisé pour la synthèse de divers produits pharmaceutiques (agents antiviraux, anti-inflammatoires) ou phytosanitaires (herbicides, insecticides). Il est également utilisé pour la synthèse de peresters tel que le perpivalate de tertiobutyle et le perpivalate de tertioamyle qui sont notamment utilisés comme initiateurs pour les polymérisations radicalaires. Les chlorures d'aroyles constituent également des intermédiares de synthèse importants, utilisés notamment pour la fabrication des peroxydes et de peresters et dans la synthèse de divers colorants, insecticides, additifs pour caoutchouc.

Les principales voies d'accès au chlorure de pivaloyle comprennent des procédés qui font réagir des réactifs classiques tels que le phosgène, le chlorure de sulfonyle, les tri- ou pentachlorures de phosphore, le chlorure de thionyle, le chlorure d'oxalyle sur l'acide pivalique ou bien encore l'oxyde de carbone, en présence de catalyseur, sur le chlorure de tertiobutyle.

Cependant, tous ces procédés représentent des technologies complexes compte tenu des réactifs mis en jeu et de la nécessité d'effectuer des traitements coûteux des produits obtenus et des effluents, rédhibitoires pour une production industrielle.

Ainsi, par exemple dans le procédé décrit par Butlerow (Justus Liebigs Ann. Chem., p. 373, 1874) qui consiste à faire réagir l'acide pivalique sur le pentachlorure de phosphore selon la réaction :

$$(CH_3)_3\ C\ COOH + PCl_5 \rightarrow (CH_3)_3\ C\ COCl + POCl_3 + HCl$$

le $POCl_3$ et le chlorure de pivaloyle obtenus ont des températures d'ébullition si proches (104-106°C) qu'il est quasiment impossible de les séparer. Aussi, cet auteur a ajouté dans le milieu réactionnel obtenu du pivalate de potassium afin de transformer $POCl_3$ en $P_2O_5$ selon la réaction :

$$3(CH_3)_3\ C\ CO_2K + 2POCl_3 \rightarrow 3(CH_3)_3\ CCOCl + P_2O_5 + 3KCl$$

D'autres auteurs (Bull. Soc. Chim. Fr., p. 350-351, 1939), à la lueur de ce procédé ont proposé de préparer directement le chlorure de pivaloyle en une seule étape par réaction du pivalate de sodium avec $POCl_3$ selon la réaction :

$$3(CH_3)_3CCO_2\ Na + 2POCl_3 \rightarrow 3(CH_3)_3\ CCOCl + P_2O_5 + 3NaCl$$

Avec un excès molaire de 25 % de pivalate de sodium, le rendement molaire en chlorure de pivaloyle n'est que de 81 % par rapport au $POCl_3$ mis en oeuvre ce qui est bien sur rédhibitoire pour un procédé industriel d'autant que le prix du pivalate de sodium est très supérieur au prix du chlorure de pivaloyle désiré.

Au lieu de $PCl_5$, on a proposé d'utiliser le trichlorure de phosphore (J. Am. Chem. Soc., 54, p. 3438-41, 1932).

Selon la réaction :

$$PCl_3 + (CH_3)_3\ C\ COOH \rightarrow (CH_3)_3\ CCOCl + H_3PO_3 + HCl$$

l'acide chlorhydrique formé est éliminé en continu et le chlorure de pivaloyle est purifié par distillation après décantation de l'acide phosphoreux qui peut être valorisé. Cependant, le rendement molaire en chlorure de pivaloyle est inférieur à 90 % par rapport à l'acide pivalique mis en oeuvre et il est très difficile d'éliminer les dernières traces d'acide phosphoreux (produit réducteur), lesquelles sont rédhibitoires pour l'utilisation du chlorure de pivaloyle dans certaines synthèses.

L'un des procédés de synthèse du chlorure de pivaloyle le plus mentionné dans la littérature est celui mettant en oeuvre le chlorure de thionyle selon la réaction :

$$(CH_3)_3\ C\ COOH + SOCl_2 \rightarrow (CH_3)_3\ C\ COCl + SO_2 + HCl$$

D'une façon générale, la réaction est effectuée en présence d'un excès molaire de 20 % à 50 % de $SOCl_2$.
Selon ces conditions, on obtient des rendements molaires en chlorure de pivaloyle distillé qui avoisinent les 90 %. L'ajout de catalyseurs tels que le DMF, la pyridine ou le N-méthylacétamide permet d'accroître la cinétique de réaction et d'améliorer la sélectivité (abaissement du pourcentage de produits secondaires tels que l'anhydride).
Cependant, ce procédé présente l'inconvénient de conduire à un chlorure de pivaloyle susceptible de contenir du soufre. En outre, dans l'éventualité où l'on utilise un catalyseur, son recyclage est difficile.
Le chlorure de pivaloyle peut être obtenu également à partir du phosgène selon la réaction :

$$(CH_3)_3CCO_2H + COCl_2 \rightarrow (CH_3)_3CCOCl + HCl + CO_2$$

ou bien encore par carbonylation du chlorure de tertiobutyle en présence de catalyseurs tels que $AlCl_3$, $FeCl_3$ selon la réaction :

$$(CH_3)_3CCl + CO \rightarrow (CH_3)_3CCOCl$$

Cependant, ces procédés présentent l'inconvénient d'utiliser des réactifs fortement toxiques, difficiles à manipuler et nécessitant l'utilisation de catalyseurs pour obtenir une bonne sélectivité et des rendements supérieurs à 90 %.
A noter que dans le cas de la carbonylation du chlorure de tertiobutyle, l'utilisation de catalyseurs est susceptible d'entraîner la formation d'impuretés ou de provoquer la rétrogradation du produit formé.
Les voies d'accès aux chlorures d'aroyles, notamment au chlorure de benzoyle comprennent également des procédés qui font réagir des réactifs classiques tels que $PCl_5$, $COCl_2$, $SOCl_2$, sur les acides aromatiques.
Plus précisément, le chlorure de benzoyle est obtenu industriellement par l'hydrolyse partielle du phénylchloroforme selon la réaction :

$$C_6H_5CCl_3 + H_2O \rightarrow C_6H_5COCl + 2HCl$$

ou par réaction de l'acide benzoïque sur le phénylchloroforme selon la réaction :

$$C_6H_5CCl_3 + C_6H_5CO_2H \rightarrow 2C_6H_5COCl + HCl$$

La production simultanée de chlorure de pivaloyle et de chlorure d'aroyle, plus précisément de chlorure de benzoyle est peu décrite dans la littérature.
Cette production simultanée de chlorures d'acides est basée sur la réaction :

$$RCOOH + C_6H_5CCl_3 \rightarrow RCOCl + C_6H_5COCl + HCl$$

qui est une réaction de chlorodeshydroxylation de RCOOH par $C_6H_5CCl_3$.
Ainsi, dans les brevets JP 57 082 336 et JP 57 165 341, on décrit un procédé de préparation en batch de chlorure de pivaloyle et de chlorure de benzoyle.
Le procédé de JP 57 165 341 consiste à faire réagir dans une première étape l'acide pivalique et le phénylchloroforme en quantité stoechiométrique à pression atmosphérique en présence de $FeCl_3$ à une température allant de 40°C à 150°C puis, après élimination de l'HCl formé, à distiller sous pression reduite le chlorure de pivaloyle. Ensuite, dans une seconde étape, après introduction d'une nouvelle charge de catalyseur, le milieu réactionnel est chauffé à une température comprise entre 40° et 160°C puis on distille sous pression réduite le chlorure de benzoyle formé.
Bien que ce procédé permet d'obtenir des rendements acceptables, compris entre 90 % et 95 % en chlorure de pivaloyle et en chlorure de benzoyle, cette façon d'opérer présente de nombreux inconvénients.
Ainsi, il est nécessaire d'éliminer la totalité du chlorure de pivaloyle avant d'effectuer la seconde étape sous risque d'effectuer la décarbonylation du chlorure de pivaloyle selon la réaction :

$$(CH_3)_3\ C\ COCl \xrightarrow[FeCl_3]{\Delta} (CH_3)_3\ C\ Cl + CO$$

Afin d'éviter cela les auteurs du brevet mentionné ont parfait la distillation en extrayant le chlorure de pivaloyle résiduel par une distillation partielle sous pression réduite du chlorure de benzoyle formé. Dans ces conditions, la fraction distillante est constituée essentiellement de chlorure de benzoyle, de quelques % de chlorure de pivaloyle et d'autres composés non identifiés.

Cette façon d'opérer présente un inconvénient important quand on sait que divers sous-produits sont susceptibles de se former suite à des réactions secondaires dont les plus importantes sont les suivantes :

- une réaction équilibrée de transhalogénation entre le chlorure de benzoyle et l'acide pivalique :

$$(CH_3)_3\ C\ COOH + C_6H_5COCl \underset{2}{\overset{1}{\rightleftharpoons}} (CH_3)_3CCOCl + C_6H_5CO_2H$$

- la réaction de l'acide benzoïque formé avec le chlorure de benzoyle pour conduire à l'anhydride benzoïque :

$$C_6H_5CO_2H + C_6H_5COCl \rightarrow (C_6H_5CO)_2O + HCl$$

Ces sous-produits formés lors de la première étape sont difficiles à éviter. Aussi, les auteurs du brevet mentionné ont, dans la seconde étape, ajouté une quantité importante de $FeCl_3$ afin de transformer les produits secondaires et, notamment l'anhydride benzoïque en présence de $C_6H_5CCl_3$ non transformé selon la réaction :

$$(C_6H_5CO)_2O + C_6H_5CCl_3 \rightarrow 3C_6H_5COCl$$

Enfin, ce procédé batch présente des opérations longues, successives, transitoires et peu productives. En outre, ce procédé est totalement silencieux sur une quelconque valorisation de l'acide chlorhydrique formé.

La demanderesse a maintenant trouvé un procédé continu de préparation de chlorure de pivaloyle et de chlorure d'aroyle (2) par réaction de l'acide pivalique sur un composé aromatique trichlorométhylé (1) selon la réaction :

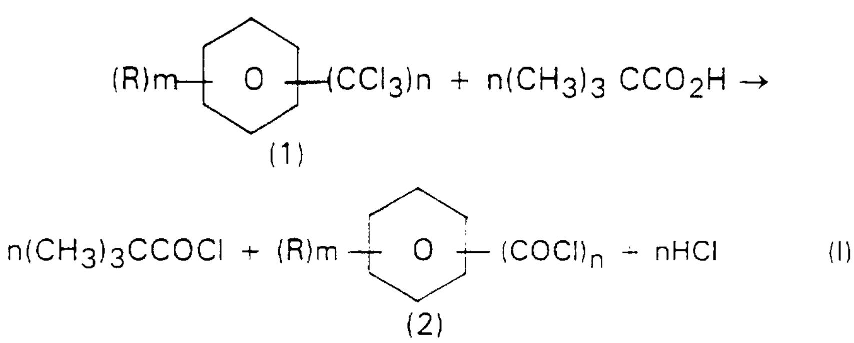

(1)

(2)

(I)

dans laquelle R représente un atome d'halogène tel que F, Cl, Br, un radical alkyle, linéaire ou ramifié, ayant un nombre de carbone allant de 1 à 4, un radical perfluoroalkyle, linéaire ou ramifié, ayant un nombre de carbone allant de 1 à 4, un radical -COCl, m=0, 1 ou 2 ; n = 1, 2 ou 3, dans laquelle les groupements $-CCl_3$ sont situés sur des atomes de carbone non-adjacents lorsque n>1 ; en présence d'au moins un catalyseur de type Friedel et Crafts, ledit procédé étant caractérisé en ce qu'il consiste :

- à introduire simultanément et en continu dans une zone de réaction l'acide pivalique, au moins un composé aromatique trichlorométhylé (1) et au moins un catalyseur de type Friedel et Crafts, et à les faire réagir sous agitation et sous pression réduite, à une température comprise entre 60°C et 180°C et, de préférence comprise entre 120°C et 150°C,

- à séparer, en continu, les réactifs non transformés des produits formés du flux gazeux sortant en tête de la zone de réaction,
- à condenser partiellement lesdits produits formés en un mélange liquide comprenant du chlorure de pivaloyle et du chlorure d'aroyle (2),
- à traiter, dans une zone de lavage dans laquelle circule à contrecourant un composé aromatique trichlorométhylé (1), le mélange gazeux non condensé restant comprenant du chlorure d'hydrogène, et
- à extraire en continu en pied de ladite zone de réaction un mélange liquide comprenant majoritairement le chlorure d'aroyle (2) formé et le catalyseur utilisé dans la zone de réaction, ledit mélange étant traité dans une zone de distillation dite "réactive" de laquelle est extrait en tête le chlorure d'aroyle (2).

Les réactifs non transformés retournent avantageusement à la zone de réaction.

Selon la présente invention, le composé aromatique trichlorométhylé (1) utilisé dans la zone de lavage peut être introduit partiellement ou en totalité dans la zone de réaction après avoir été utilisé dans la zone de lavage. De préférence, on l'introduira en totalité dans la zone de réaction de façon à recycler avantageusement le chlorure de pivaloyle absorbé par le composé aromatique trichlorométhylé (I) dans la zone de lavage.

Selon la présente invention, le mélange extrait en pied de zone de réaction, comprenant majoritairement du chlorure d'aroyle et, également du composé aromatique trichlorométhylé (1) non transformé, du catalyseur, de l'anhydride d'aroyle et éventuellement de faibles quantités de produits secondaires est traité dans une zone de distillation dite "réactive" sous pression réduite à une température au moins égale à 120°C. On peut utiliser en outre une quantité supplémentaire de catalyseur de type Friedel et Crafts, identique ou différent de celui utilisé dans la zone de réaction. On récupère en tête la partie complémentaire du chlorure d'aroyle formé dans le procédé. En pied, on récupère des lourds qui sont détruits notamment par incinération.

La réaction (I) est effectuée avec un rapport molaire :

$$\frac{\text{composé aromatique trichlorométhylé (1)}}{\text{acide pivalique}}$$

allant de 0,90 n à 1,10 n, et, de préférence, compris entre 1n et 1,03n.

Selon la présente invention, on opère dans la zone de réaction sous une pression réduite au plus égale à $5.10^4$ Pa (500 mbars) et, de préférence comprise entre $10^4$ Pa (100 mbars) et $4.10^4$ Pa (400 mbars).

Par catalyseur de type Friedel et Crafts, on désigne présentement un acide de Lewis ou un acide de Brönsted.

A titre d'illustration d'acide de Lewis utilisable selon l'invention, on citera $FeCl_3$, $ZnCl_2$, $SnCl_4$, $AlCl_3$, $SbCl_5$, $CoCl_2$, $BF_3$...

De préférence, on utilisera $FeCl_3$.

A titre d'illustration d'acide de Brönsted utilisable selon la présente invention, on citera l'acide sulfurique, l'acide phosphorique, les acides polyphosphoriques, l'acide pyrosulfurique, l'acide fluorosulfonique, l'acide chlorosulfonique. De préférence, on utilisera l'acide sulfurique.

Ces catalyseurs de type Friedel et Crafts peuvent être introduits dans la zone de réaction tels quels ou sous forme de solutions aqueuses ou en solution dans un des réactifs ou bien encore en solution dans du chlorure de pivaloyle ou de chlorure d'aroyle.

Selon la présente invention, on utilisera une quantité molaire d'acide de Lewis pur comprise entre 0,01 % et 1 % et, de préférence, comprise entre 0,05 % et 0,2 % par rapport à la quantité d'acide pivalique mis en oeuvre.

Selon la présente invention, on utilisera une quantité molaire d'acide de Brönsted pur comprise entre 0,1 % et 5 % et, de préférence, comprise entre 0,5 % et 2 %, par rapport à la quantité d'acide pivalique mis en oeuvre.

On ne sortirait pas du cadre de l'invention si le composé aromatique trichlorométhylé circulant à contre courant dans la zone de lavage était différent de celui introduit dans la zone de réaction.

Le chlorure d'hydrogène pur sortant en tête de ladite zone de lavage est ensuite avantageusement absorbé dans de l'eau pour conduire à des solutions aqueuses d'HCl commerciales.

Le mélange liquide extrait en tête de la zone de réaction constitué par un mélange de chlorure de pivaloyle et de chlorure d'aroyle est avantageusement soumis à une distillation sous pression réduite qui permet d'effectuer la séparation des différents chlorures formés.

A titre d'illustration de composés aromatiques trichlorométhylés (1) utilisables selon la présente invention, on citera :

- le trichlorométhylbenzène ou phénylchloroforme,

- les 2-chloro, 3-chloro et 4-chloro-1-trichlorométhylbenzènes,
- les 1,3- et 1,4-bis(trichloromethyl)benzènes,
- le 4-fluoro-1-trichlorométhylbenzène,
- le 3,4-dichloro-1-trichlorométhylbenzène,
- le 4-trifluorométhyl-1-trichlorométhylbenzène.

Le procédé de l'invention s'applique tout particulièrement à la préparation du chlorure de pivaloyle et du chlorure de benzoyle à partir de l'acide pivalique et du phénylchloroforme.

Le procédé présente l'avantage de conduire à des chlorures d'acides de pureté élevée et d'une qualité constante avec des hauts rendements, supérieurs à 96 %, une conversion totale des réactifs et une productivité élevée. En outre, le chlorure d'hydrogène formé est directement valorisé.

Ce procédé présente également l'avantage de générer peu de produits secondaires et surtout ne produit pas d'effluents gazeux.

L'exemple qui suit illustre l'invention.

On introduit en continu, sous agitation et sous une pression de $1,33.10^4$ Pa (133 mbars), 130 kg/h d'acide pivalique avec 0,5 kg/h d'une solution aqueuse à 40 % en poids de $FeCl_3$ et 285,5 kg/h d'un flux de phénylchloroforme contenant 35,5 kg/h de chlorure de pivaloyle dans un réacteur vitrifié de 2,5 $m^3$ maintenu à 135°C, en régime stabilisé, contenant un mélange comprenant du chlorure de benzoyle, du phénylchloroforme et de l'anhydride benzoïque.

Dans ces conditions, le chlorure de pivaloyle est immédiatement formé et vaporisé ainsi qu'une partie du chlorure de benzoyle.

Les produits gazeux de la réaction sont extraits en continu en tête du réacteur et sont entraînés dans une petite colonne de séparation équipée d'un garnissage structuré de 6 plateaux théoriques où ils sont séparés des réactifs non tranformés. Les réactifs non transformés retournent dans le réacteur.

Après condensation du mélange de produits gazeux, on extrait en continu,

d'une part un mélange liquide constitué de 147,7 kg/h de chlorure de pivaloyle, de 82,8 kg/h de chlorure de benzoyle et de 0,9 kg/h de chlorure de tertiobutyle qui est envoyé dans un bac de stockage pour être soumis ultérieurement à une distillation qui permet de récupérer le chlorure de pivaloyle ayant une pureté supérieure à 99,5 %,

et d'autre part, un flux gazeux constitué de 47,8 kg/h de chlorure d'hydrogène (HClgaz), de 35,6 kg/h de chlorure de pivaloyle et de 2,7 kg/h de chlorure de tertiobutyle, qui est aspiré par un éjecteur liquide et envoyé dans une colonne de lavage garnie de 9 plateaux théoriques, dans laquelle le chlorure d'hydrogène est lavé à contre courant par 200 kg/h de phénylchloroforme à 10°C.

En tête de cette colonne de lavage, on récupère 47,5 kg/h d'HCl gaz qui sont envoyés sur un absorbeur isotherme à film tombant où ils sont absorbés par 95,5 kg/h d'eau pour former 144,9 kg/h d'une solution aqueuse à 33 % en poids d'HCl.

En pied de la zone de lavage, le phénylchloroforme ayant absorbé les composés organiques contenus dans le flux gazeux introduit dans ladite colonne de lavage est envoyé dans le réacteur ; il forme une partie des 285,6 kg/h du phénylchloroforme mise en oeuvre.

En pied de réacteur, on extrait en continu 102,3 kg/h d'un mélange liquide, contenant 55,6 kg de chlorure de benzoyle,

16,9 kg de phénylchloroforme,
21,3 kg d'anhydride benzoïque,
2,8 kg de chlorure de pivaloyle,
1,4 kg d'acide benzoïque, du catalyseur ainsi que des résidus lourds liés aux impuretés du phénylchloroforme mis en oeuvre.

Ce mélange liquide est évacué dans un bac de stockage puis est soumis à une distillation réactive dans une colonne de 20 plateaux théoriques, sous pression réduite, à une température comprise entre 120°C et 150°C, et en présence de 0,1 % en poids de $FeCl_3$ par rapport au poids du mélange. Il sort en tête de cette colonne, le chlorure de pivaloyle résiduel puis le chlrorue de benzoyle avec une pureté supérieure à 99,9 %.

Les produits lourds sont éliminés par incinération.

Après les distillations, les rendements en chlorure de pivaloyle et en chlorure de benzoyle sont respectivement supérieurs à 96,8 % et 97 %. L'acide chlorhydrique obtenu dans ce procédé est une solution aqueuse à 33 % d'HCl de qualité commerciale.

**Revendications**

1. Procédé continu de préparation de chlorure de pivaloyle et de chlorure d'aroyle (2) par réaction de l'acide pivalique sur un composé aromatique trichlorométhylé (1) selon la réaction :

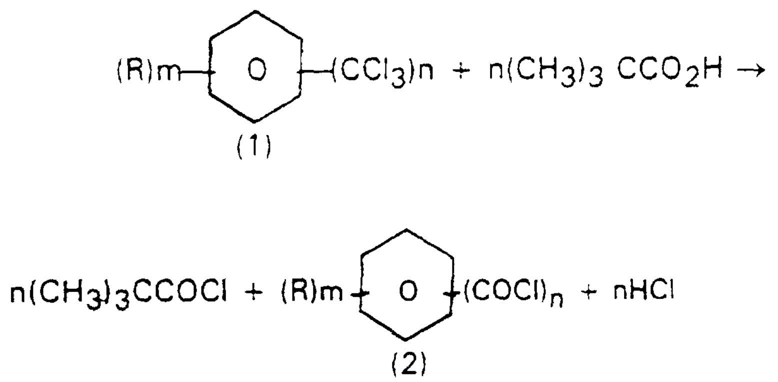

(I)

dans laquelle R représente un atome d'halogène tel que F, Cl, Br, un radical alkyle, linéaire ou ramifié, ayant un nombre de carbone allant de 1 à 4, un radical perfluoroalkyle, linéaire ou ramifié, ayant un nombre de carbone allant de 1 à 4, un radical -COCl, m=O, 1 ou 2, n=1, 2 ou 3 et que les groupements $-CCl_3$ sont situés sur des atomes de carbone non-adjacents lorsque n > 1 ; en présence d'au moins un catalyseur de type Friedel et Crafts, **caractérisé en ce qu'**il consiste :

- à introduire simultanément et en continu dans une zone de réaction l'acide pivalique, au moins un composé aromatique trichlorométhylé (1) et au moins un catalyseur de type Friedel et Crafts, et à les faire réagir sous agitation et sous pression réduite, à une température comprise entre 60°C et 180°C,
- à séparer, en continu, les réactifs non transformés des produits formés du flux gazeux sortant en tête de la zone de réaction,
- à condenser partiellement lesdits produits formés en un mélange liquide comprenant du chlorure de pivaloyle et chlorure de benzoyle,
- à traiter, dans une zone de lavage dans laquelle circule à contrecourant un composé aromatique trichlorométhylé (1), le mélange gazeux non condensé restant comprenant du chlorure d'hydrogène, et
- à extraire, en continu, en pied de ladite zone de réaction un mélange liquide comprenant majoritairement le chlorure d'aroyle (2) formé et le catalyseur utilisé dans la zone de réaction, ledit mélange étant traité dans une zone de distillation dite "réactive" de laquelle est extrait en tête le chlorure d'aroyle (2).

2. Procédé selon la revendication 1, **caractérisé en ce que** la distillation réactive est effectuée en présence d'une quantité supplémentaire de catalyseur de type Friedel et Crafts.

3. Procédé selon la revendication 1, **caractérisé en ce que** la température dans la zone de réaction est comprise entre 120°C et 150°C.

4. Procédé selon l'une des revendications 1 ou 3, **caractérisé en ce que** l'on opère dans la zone de réaction sous une pression réduite au plus égale à $5.10^4$ Pa (500 mbars).

5. Procédé selon la revendication 4, **caractérisé en ce que** l'on opère dans la zone de réaction sous une pression réduite comprise entre $10^4$ Pa (100 mbars) et $4.10^4$ Pa (400 mbars).

6. Procédé selon l'une quelconque des revendications 1 et 3 à 5, **caractérisé en ce que** la réaction (I) est effectuée avec rapport molaire :

$$\frac{\text{composé aromatique trichlorométhyle (1)}}{\text{acide pivalique}}$$

allant de 0,90 n à 1,10 n et, de préférence, compris entre 1n et 1,03n.

7. Procédé selon l'une quelconque des revendications 1 à 6, **caractérisé en ce que** le catalyseur de type Friedel et Crafts est un acide de Lewis.

8. Procédé selon la revendication 7, **caractérisé en ce que** l'acide de Lewis est $FeCl_3$.

9. Procédé selon l'une des revendications 7 ou 8, **caractérisé en ce que** l'on utilise une quantité molaire d'acide de Lewis, comprise entre 0,01 % et 1 %, et de préférence, comprise entre 0,05 % et 0,2 % par rapport à la quantité d'acide pivalique mis en oeuvre.

10. Procédé selon l'une quelconque des revendications 1 à 6, **caractérisé en ce que** le catalyseur de type Friedel et Crafts est un acide de Brönsted.

11. Procédé selon la revendication 10, **caractérisé en ce que** l'acide de Brönsted est l'acide sulfurique.

12. Procédé selon l'une quelconque des revendications 10 ou 11, **caractérisé en ce que** l'on utilise une quantité molaire d'acide de Brönsted comprise entre 0,1 % et 5 % par rapport à la quantité d'acide pivalique mis en oeuvre.

13. Procédé selon l'une quelconque des revendications 1 à 12, **caractérisé en ce que** le composé trichlorométhylé (1) utilisé dans la zone de lavage est introduit en totalité dans la zone de réaction.

14. Procédé selon l'une quelconque des revendications 1 à 13, **caractérisé en ce que** le composé aromatique trichlorométhylé (1) est le trichlorométhylbenzène (ou phénylchloroforme).

## Patentansprüche

1. , Kontinuierliches Verfahren zur Herstellung von Pivaloyl- und Aroylchlorid (2) durch Einwirkung von Pivalinsäure auf eine trichlormethylierte aromatische Verbindung (1) nach der Reaktion:

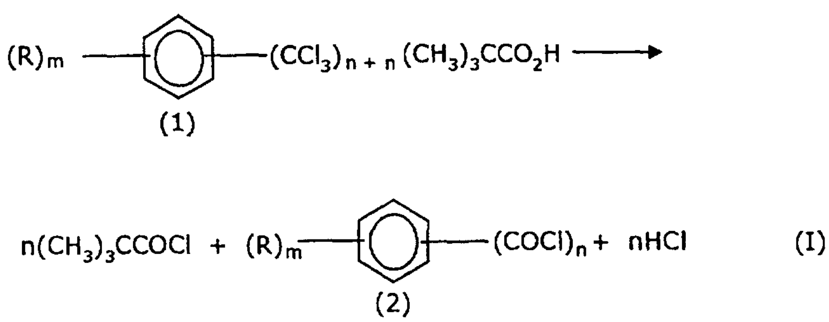
(1)
(2)
(I)

in der R ein Halogenatom wie F, Cl, Br, einen gerad- oder verzweigtkettigen Alkylrest mit 1 bis 4 Kohlenstoffatomen, einen gerad- oder verzweigtkettigen Perfluoralkylrest mit 1 bis 4 Kohlenstoffatomen oder einen -COCl-Rest bedeutet, m = 0, 1 oder 2, n = 1, 2 oder 3 ist, wobei die $-CCl_3$-Gruppen sich an nicht benachbarten Kohlenstoffatomen befinden, wenn n > 1 ist; in Gegenwart mindestens eines Friedel-Cafts-Katalysators, **dadurch gekennzeichnet, daß** man:

- einer Reaktionszone gleichzeitig oder kontinuierlich Pivalinsäure, wenigstens eine trichlormethylierte aromatische Verbindung (1) und mindestens einen Friedel-Crafts-Katalysator zugibt und diese unter Rühren bei vermindertem Druck bei einer Temperatur zwischen 60 °C und 180 °C reagieren läßt,
- kontinuierlich die nicht umgesetzten Reagenzien von den gebildeten Produkten aus dem die Reaktionszone am Kopf verlassenden Gasstrom trennt,
- die genannten gebildeten Produkte teilweise zu einem flüssigen Gemisch kondensiert, das Pivaloylchlorid und Benzoylchlorid enthält,
- das zurückgebliebene, nicht kondensierte Chlorwasserstoff enthaltende Gemisch in einer Waschzone behandelt, in der eine trichlormethylierte aromatische Verbindung (1) im Gegenstrom zirkuliert, und
- kontinuierlich am Fuß der Reaktionszone ein flüssiges Gemisch abzieht, das überwiegend das gebildete Aroylchlorid (2) und den in der Reaktionszone verwendeten Katalysator enthält, wobei das Gemisch in einer reaktiven Destillationszone behandelt wird, aus der am Kopf das Aroylchlorid (2) abgezogen wird.

2. Verfahren nach Anspruch 1, **dadurch gekennzeichnet, daß** die reaktive Destillation in Gegenwart einer zusätzlichen Menge des Friedel-Crafts-Katalysators durchgeführt wird.

3. Verfahren nach Anspruch 1, **dadurch gekennzeichnet, daß** die Temperatur in der Reaktionszone zwischen 120 °C und 150 °C liegt.

4. Verfahren nach einem der Ansprüche 1 oder 3, **dadurch gekennzeichnet, daß** man in der Reaktionszone bei einem verminderten Druck bis höchstens gleich $5 \cdot 10^4$ Pa (500 mbar) arbeitet.

5. Verfahren nach Anspruch 4, **dadurch gekennzeichnet, daß** man in der Reaktionszone bei einem verminderten Druck zwischen $10^4$ Pa (100 mbar) und $4 \cdot 10^4$ Pa (400 mbar) arbeitet.

6. Verfahren nach einem der Ansprüche 1, 3 bis 5, **dadurch gekennzeichnet, daß** die Reaktion mit einem Molverhältnis

$$\frac{\text{trichlormethylierte aromatische Verbindung (1)}}{\text{Pivalinsäure}}$$

von 0,90 n bis 1,10 n und vorzugsweise zwischen 1 n und 1,03 n durchgeführt wird.

7. Verfahren nach einem der Ansprüche 1 bis 6, **dadurch gekennzeichnet, daß** der Friedel-Crafts-Katalysator eine Lewis-Säure ist.

8. Verfahren nach Anspruch 7, **dadurch gekennzeichnet, daß** die Lewis-Säure $FeCl_3$ ist.

9. Verfahren nach einem der Ansprüche 7 oder 8, **dadurch gekennzeichnet, daß** man eine molare Menge der Lewis-Säure zwischen 0,01 % und 1% und vorzugsweise zwischen 0,05 % und 0,2 %, bezogen auf die Menge der eingesetzten Pivalinsäure, verwendet.

10. Verfahren nach einem der Ansprüche 1 bis 6, **dadurch gekennzeichnet, daß** der Friedel-Crafts-Katalysator eine Brönsted-Säure ist.

11. Verfahren nach Anspruch 10, **dadurch gekennzeichnet, daß** die Brönsted-Säure Schwefelsäure ist.

12. Verfahren nach einem der Ansprüche 10 oder 11, **dadurch gekennzeichnet, daß** man eine molare Menge der Brönsted-Säure zwischen 0,1 % und 5 %, bezogen auf die Menge der eingesetzten Pivalinsäure, verwendet.

13. Verfahren nach einem der Ansprüche 1 bis 12, **dadurch gekennzeichnet, daß** die in der Waschzone verwendete trichlormethylierte Verbindung (1) vollständig in der Reaktionszone zugegeben wird.

14. Verfahren nach einem der Ansprüche 1 bis 13, **dadurch gekennzeichnet, daß** die aromatische trichlormethylierte Verbindung Trichlormethylbenzol (oder Phenylchloroform) ist.

**Claims**

1. Continuous process for the preparation of pivaloyl chloride and of aroyl chloride (2) by reaction of pivalic acid with a trichloromethylated aromatic compound (1) according to the reaction:

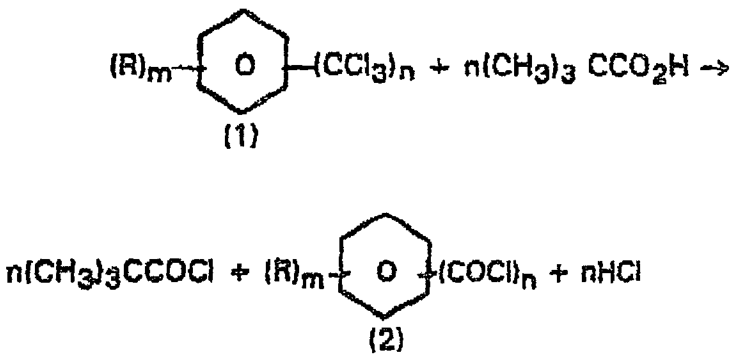

(1) (2) (I)

in which R represents a halogen atom, such as F, Cl or Br, a linear or branched alkyl radical having a carbon number ranging from 1 to 4, a linear or branched perfluoroalkyl radical having a carbon number ranging from 1 to 4 or a -COCl radical, m = 0, 1 or 2 and n = 1, 2 or 3 and in which the $-CCl_3$ groups are situated on non-adjacent carbon atoms when n > 1, in the presence of at least one catalyst of Friedel-Crafts type, **characterized in that** it consists:

- in simultaneously and continuously introducing pivalic acid, at least one trichloromethylated aromatic compound (1) and at least one catalyst of Friedel-Crafts type into a reaction region and in reacting them with stirring and under reduced pressure at a temperature of between 60°C and 180°C,
- in continuously separating the unconverted reactants from the products formed of the gas flow exiting at the top of the reaction region,
- in partially condensing the said products formed into a liquid mixture comprising pivaloyl chloride and aroyl chloride (2),
- in treating, in a washing region in which a trichloromethylated aromatic compound (1) moves countercurrentwise, the remaining uncondensed gaseous mixture comprising hydrogen chloride, and
- in continuously extracting, at the bottom of the said reaction region, a liquid mixture mainly comprising the aroyl chloride (2) formed and the catalyst used in the reaction region, the said mixture being treated in a so-called "reactive" distillation region, from which is extracted, at the top, the aroyl chloride (2).

**2.** Process according to Claim 1, **characterized in that** the reactive distillation is carried out in the presence of an additional amount of catalyst of Friedel-Crafts type.

**3.** Process according to Claim 1, **characterized in that** the temperature in the reaction region is between 120°C and 150°C.

**4.** Process according to either of Claims 1 and 3, **characterized in that** the reaction is carried out in the reaction region under a reduced pressure at most equal to $5 \times 10^4$ Pa (500 mbar).

**5.** Process according to Claim 4, **characterized in that** the reaction is carried out in the reaction region under a reduced pressure of between $10^4$ Pa (100 mbar) and $4 \times 10^4$ Pa (400 mbar).

**6.** Process according to any one of Claims 1 and 3 to 5, **characterized in that** the reaction (I) is carried out with a molar ratio:

$$\frac{\text{trichloromethylated aromatic compound (1)}}{\text{pivalic acid}}$$

ranging from 0.90n to 1.10n and preferably of between In and 1.03n.

**7.** Process according to any one of Claims 1 to 6, **characterized in that** the catalyst of Friedel-Crafts type is a Lewis acid.

**8.** Process according to Claim 7, **characterized in that** the Lewis acid is $FeCl_3$.

9. Process according to either of Claims 7 and 8, **characterized in that** use is made of a molar amount of Lewis acid of between 0.01% and 1% and preferably of between 0.05% and 0.2%, with respect to the amount of pivalic acid employed.

10. Process according to any one of Claims 1 to 6, **characterized in that** the catalyst of Friedel-Crafts type is a Bronsted acid.

11. Process according to Claim 10, **characterized in that** the Brönsted acid is sulphuric acid.

12. Process according to either one of Claims 10 and 11, **characterized in that** use is made of a molar amount of Brönsted acid of between 0,1% and 5%, with respect to the amount of pivalic acid employed.

13. Process according to any one of Claims 1 to 12, **characterized in that** the trichloromethylated compound (1) used in the washing region is completely introduced into the reaction region.

14. Process according to any one of Claims 1 to 13, **characterized in that** the trichloromethylated aromatic compound (1) is trichloromethylbenzene (or phenylchloroform).